# EUROPÄISCHE PATENTSCHRIFT

(11) **EP 2 070 544 B1**
(45) Veröffentlichungstag und Bekanntmachung des Hinweises auf die Patenterteilung: **05.06.2013**
(21) Anmeldenummer: 08020220.3
(22) Anmeldetag: 20.11.2008
(51) Int. Cl.: A61K 36/185, A61K 36/33, A61K 36/258, A61K 36/34, A61K 36/56, A61K 36/81, A61K 36/22, A61K 41/00, A61K 33/04, A61K 33/18, A61K 33/30, A61P 25/34

(54) **Mittel zur Raucherentwöhnung**
Means of withdrawal from smoking
Moyen destiné au sevrage tabagique

(30) Priorität: 13.12.2007 DE 202007017543 U
(43) Veröffentlichungstag der Anmeldung: 17.06.2009
(73) Patentinhaber: Gross, Marianna, 66557 IIlingen (DE)
(72) Erfinder: Gross, Marianna, 66557 IIlingen (DE)
(74) Vertreter: Vièl, Christof

(56) Entgegenhaltungen:
- DE-U1-202008 007 923
- US-A1- 2005 100 513
- US-A1- 2006 062 835
- DATABASE WPI Week 200049 Thomson Scientific, London, GB; AN 2000-541666 XP002567273 & RU 2 143 274 C1 (DOKTOR N CO LTD) 27. Dezember 1999 (1999-12-27)

## Beschreibung

Die Erfindung betrifft ein Mittel zur Raucherentwöhnung.

Es ist bekannt, daß Rauchen schnell zu Suchterscheinungen führt. Viele Raucher, die das Rauchen aufgeben möchten, sind daher mit Entzugsproblemen konfrontiert, welche dazu führen, daß in vielen Fällen der Raucher rückfällig wird.

Es sind Nikotinpflaster auf dem Markt, die eine Nikotinaufnahme über die Haut ermöglichen. Diese lösen jedoch weder das Problem der Nikotinabhängigkeit noch bieten sie einen Ersatz für die Geste des Rauchens, so daß auch bei Verwendung von Nikotinpflastern häufig eine dauerhafte Raucherentwöhnung scheitert.

Die DE 20 2008 007 923 U1 beschreibt ein Suppositorium, das zumindest Nicotiana tabacum enthält.

Aus der US 2006/062835 A1 geht ein Verfahren zur Nikotinentwöhnung hervor, bei dem in einer ersten Phase ein Druckpad auf das Ohr des Patienten aufgebracht wird, dann der Patient in einer zweiten Phasen oral ein Entzugsmittel einnimmt, in einer dritten Phase, die sich mit der ersten Phase überlappt, eine streßreduzierende Substanz oral zu sich nimmt und in einer vierten Phase diese Behandlungsschritte fortgesetzt werden.

Die US 2005/100513 A1 beschreibt ebenfalls ein Verfahren zur Nikotinentwöhnung, bei der gleichzeitig zwei homöopathische Substanzen verabreicht werden, wobei die erste Substanz der Nikotinsucht entgegenwirkt und die zweite zur Entgiftung des Körpers dient.

In der RU 2 143 274 C1 wird schließlich ein homöopathisches Mittel zur Raucherentwöhnung beschrieben, das in Form von Zuckerkügelchen ("sugar granules") verabreicht wird.

Aufgabe der Erfindung ist es daher, ein Mittel zur Raucherentwöhnung zu schaffen, mit dem eine dauerhafte Raucherentwöhnung in der Mehrzahl der Fälle möglich ist.

Diese Aufgabe wird erfindungsgemäß dadurch gelöst, daß das Mittel als homöopathisches Mittel zur Injektion ausgebildet ist, das 1,5 bis 2 Gew.-% Passiflora incarnata D30, 0,75 bis 1,25 Gew.-% Rhus toxicodendron D60, 0,75 bis 1,25 Gew.-% Ignatia D30, 1 bis 1,5 Gew.-% Cactus D30, 0,25 bis 0,75 Gew.-% Panax Ginseng D30, 0,75 bis 1,25 Gew.-% Pulsatilla D23, 1,5 bis 2 Gew.-% Lobelia inflata D60 0,5 bis 1 Gew.-% Stramonium und 1,5 bis 2,5 Gew.-% Nux Moschata D30 enthält.

Wie allgemein üblich, ist die jeweilige Potenzierung mit dem Buchstaben D sowie der entsprechenden Potenzierungsstufe bezeichnet, so daß D30 beispielsweise für eine Verdünnung von 1 : 10³⁰ steht. Das Verdünnungsmittel ist Wasser. Es handelt sich also um wässrige Lösungen der Wirkstoffe.

Mit einer einzigen Injektion von 2 ml dieses Mittels ins Ohrläppchen ist es bei einer hohen Erfolgsquote möglich, eine sofortige Raucherentwöhnung zu erreichen. Überraschenderweise treten auch während der ersten 24 Stunden in der Regel keine Entzugserscheinungen auf und es wird in der Mehrzahl der Fälle eine langfristige Raucherentwöhnung erreicht.

In seltenen Fällen ist es erforderlich, eine zweite Injektion vorzunehmen, um eine vollständige Raucherentwöhnung zu erreichen. Hierbei hängt - wie bei jeder Raucherentwöhnung - der Erfolg selbstverständlich von dem Willen des Rauchers ab, das Rauchen aufzugeben.

Es liegt im Rahmen der Erfindung, daß das homöopathische Mittel weiterhin isotonische NaCl-Lösung und/oder Wasser enthält.

Eine Weiterbildung der Erfindung besteht darin, daß das homöopathische Mittel 1 bis 1,5 Gew.-% Robinia pseudoacacia D30 enthält.

Ebenfalls kann vorgesehen sein, daß das homöopathische Mittel 1,5 bis 2 Gew.-% Zincum metallicum D26 enthält.

Es liegt auch im Rahmen der Erfindung, daß das homöopathische Mittel 0,75 bis 1,25 Gew.-% Sulphur D6 enthält.

Bei einer Ausbildung der Erfindung ist es vorgesehen, daß das homöopathische Mittel 0,75 bis 1,25 Gew.-% Iodum D30 enthält.

Weiterhin ist vorgesehen, daß das homöopathische Mittel 0,75 bis 1,25 Gew.-% Chamomilla D30 enthält.

Erfindungsgemäß ist auch vorgesehen, daß das homöopathische Mittel 1 bis 2 Gew.-% Phosphorus D28 enthält.

Zur Erfindung gehörig ist weiterhin, daß das homöopathische Mittel 0,5 bis 1 Gew.-% Nux vomica D30 enthält.

Schließlich liegt es auch im Rahmen der Erfindung, daß das homöopathische Mittel 1 bis 1,5 Gew.-% Agnus Cactus D4 enthält.

Nachfolgend wird die Erfindung anhand von Ausführungsbeispielen näher erläutert.

### Beispiel 1:

Folgende Substanzen werden mit 80 g destilliertem Wasser gemischt

| | |
|---|---|
| Passiflora incarnata D30 | 1,5 g |
| Rhus toxicodendron D60 | 0,8 g |
| Ignatia D30 | 1 g |
| Zincum metallicum D26 | 1,8 g |
| Cactus D30 | 1,1 g |
| Sulphur D6 | 0,9 g |
| Panax Ginseng D30 | 0,4 g |
| Pulsatilla D23 | 1,1 g |
| Iodum D30 | 0,9 g |
| Lobelia inflata D60 | 1,6 g |
| Stramonium D30 | 1,1 g |
| Nux Moschata D30 | 1,8 g |
| Agnus Cactus D4 | 1,1 g |

2 ml dieser Mischung werden ins Ohrläppchen des Patienten injiziert.

### Beispiel 2:

Folgende Substanzen werden mit 80 g isotonischer NaCl-Lösung gemischt

| | |
|---|---|
| Passiflora incarnata D30 | 1,5 g |
| Rhus toxicodendron D60 | 0,8 g |
| Robinia pseudoacacia D30 | 1,1 g |
| Ignatia D30 | 1 g |
| Zincum metallicum D26 | 1,8 g |
| Cactus D30 | 1,1 g |
| Sulphur D6 | 0,9 g |
| Panax Ginseng D30 | 0,4 g |
| Pulsatilla D23 | 1,1 g |
| Lobelia inflata D60 | 1,6 g |
| Chamomilla D30 | 0,9 g |
| Phosphorus D28 | 1,4 g |
| Stramonium D30 | 1,1 g |
| Nux vomica D30 | 0,7 g |
| Nux Moschata D30 | 1,8 g |

2 ml dieser Mischung werden in den äußeren Ohrrand des Patienten injiziert.

## Patentansprüche

1. Mittel zur Raucherentwöhnung, **dadurch gekennzeichnet, daß** das Mittel als homöopathisches Mittel zur Injektion ausgebildet ist, das 1,5 bis 2 Gew.-% Passiflora incarnata D30, 0,75 bis 1,25 Gew.-% Rhus toxicodendron D60, 0,75 bis 1,25 Gew.-% Ignatia D30, 1 bis 1,5 Gew.-% Cactus D30, 0,25 bis 0,75 Gew.-% Panax Ginseng D30, 0,75 bis 1,25 Gew.-% Pulsatilla D23, 1,5 bis 2 Gew.-% Lobelia inflata D60 0,5 bis 1 Gew.-% Stramonium und 1,5 bis 2,5 Gew.-% Nux Moschata D30 enthält.

2. Mittel gemäß Anspruch 1, **dadurch gekennzeichnet, daß** das homöopathische Mittel weiterhin isotonische NaCl-Lösung und/oder Wasser enthält.

3. Mittel gemäß Anspruch 1 oder 2, **dadurch gekennzeichnet, daß** das homöopathische Mittel 1 bis 1,5 Gew.% Robinia pseudoacacia D30 enthält.

4. Mittel gemäß einem der Ansprüche 1 bis 3, **dadurch gekennzeichnet, daß** das homöopathische Mittel 1,5 bis 2 Gew.-% Zincum metallicum D26 enthält.

5. Mittel gemäß einem der Ansprüche 1 bis 4, **dadurch gekennzeichnet, daß** das homöopathische Mittel 0,75 bis 1,25 Gew.-% Sulphur D6 enthält.

6. Mittel gemäß einem der Ansprüche 1 bis 5, **dadurch gekennzeichnet, daß** das homöopathische Mittel 0,75 bis 1,25 Gew.% Iodum D30 enthält.

7. Mittel gemäß einem der Ansprüche 1 bis 6, **dadurch gekennzeichnet, daß** das homöopathische Mittel 0,75 bis 1,25 Gew.% Chamomilla D30 enthält.

8. Mittel gemäß einem der Ansprüche 1 bis 7, **dadurch gekennzeichnet, daß** das homöopathische Mittel 1 bis 2 Gew.-% Phosphorus D28 enthält.

9. Mittel gemäß einem der Ansprüche 1 bis 8, **dadurch gekennzeichnet, daß** das homöopathische Mittel 0,5 bis 1 Gew.-% Nux vomica D30 enthält.

10. Mittel gemäß einem der Ansprüche 1 bis 9, **dadurch gekennzeichnet, daß** das homöopathische Mittel 1 bis 1,5 Gew.-% Agnus Cactus D4 enthält.

## Claims

1. Smoking cessation agent, **characterised in that** the agent is configured as a homeopathic injection preparation containing 1.5 to 2 wt. % Passiflora incarnata D30, 0.75 to 1.25 wt. % Rhus toxicodendron D60, 0.75 to 1.25 wt. % Ignatia D30, 1 to 1.5 wt. % Cactus D30, 0.25 to 0.75 wt. % Panax ginseng D30, 0.75 to 1.25 wt. % Pulsatilla D23, 1.5 to 2 wt. % Lobelia inflata D60, 0.5 to 1 wt. % Stramonium and 1.5 to 2.5 wt. % Nux moschata D30.

2. Agent according to claim 1, **characterised in that** the homeopathic agent also contains isotonic NaCl solution and/or water.

3. Agent according to claim 1 or 2, **characterised in that** the homeopathic agent contains 1 to 1.5 wt. % Robinia pseudoacacia D30.

4. Agent according to one of claims 1 to 3, **characterised in that** the homeopathic agent contains 1.5 to 2 wt. % Zincum metallicum D26.

5. Agent according to one of claims 1 to 4, **characterised in that** the homeopathic agent contains 0.75 to 1.25 wt. % Sulphur D6.

6. Agent according to one of claims 1 to 5, **characterised in that** the homeopathic agent contains 0.75 to 1.25 wt. % Iodum D30.

7. Agent according to one of claims 1 to 6, **characterised in that** the homeopathic agent contains 0.75 to 1.25 wt. % Chamomilla D30.

8. Agent according to one of claims 1 to 7, **characterised in that** the homeopathic agent contains 1 to 2 wt. % Phosphorus D28.

9. Agent according to one of claims 1 to 8, **characterised in that** the homeopathic agent contains 0.5 to 1 wt. % Nux vomica D30.

10. Agent according to one of claims 1 to 9, **characterised in that** the homeopathic agent contains 1 to 1.5 wt. % Agnus cactus D4.

## Revendications

1. Moyens destinés au sevrage tabagique **caractérisés en ce que** les moyens sont conçus comme un produit homéopathique pour injection qui contient 1,5 à 2 % en poids de Passiflora incarnata D30, 0,75 à 1,25 % en poids de Rhus toxicodendron D60, 0,75 à 1,25 % en poids d'Ignatia D30, 1 à 1,5 % en poids de Cactus D30, 0,25 à 0,75 % en poids de Panax Ginseng D30, 0,75 à 1,25 % en poids de Pulsatilla D23, 1,5 à 2 % en poids de Lobelia inflata D60, 0,5 à 1 % en poids de Stramonium et 1,5 à 2,5 % en poids de Nux Moschata D30.

2. Moyens selon la revendication 1, **caractérisés en ce que** le produit homéopathique contient en outre une solution isotonique de NaCl et/ou de l'eau.

3. Moyens selon la revendication 1 ou 2, **caractérisés en ce que** le produit homéopathique contient 1 à 1,5 % en poids de Robinia pseudoacacia D30.

4. Moyens selon l'une des revendications 1 à 3, **caractérisés en ce que** le produit homéopathique contient 1,5 à 2 % en poids de Zincum metallicum D26.

5. Moyens selon l'une des revendications 1 à 4, **caractérisés en ce que** le produit homéopathique contient 0,75 à 1,25 % en poids de Sulphur D6.

6. Moyens selon l'une des revendications 1 à 5, **caractérisés en ce que** le produit homéopathique contient 0,75 à 1,25 % en poids d'Iodum D30.

7. Moyens selon l'une des revendications 1 à 6, **caractérisés en ce que** le produit homéopathique contient 0,75 à 1,25 % en poids de Chamomilla D30.

8. Moyens selon l'une des revendications 1 à 7, **caractérisés en ce que** le produit homéopathique contient 1 à 2 % en poids de Phosphorus D28.

9. Moyens selon l'une des revendications 1 à 8, **caractérisés en ce que** le produit homéopathique contient 0,5 à 1 % en poids de Nux vomica D30.

10. Moyens selon l'une des revendications 1 à 9, **caractérisés en ce que** le produit homéopathique contient 1 à 1,5 % en poids de Agnus Cactus D4.
